(19) Europäisches Patentamt
European Patent Office
Office européen des brevets

(11) Publication number: 0 439 968 A1

# (12) EUROPEAN PATENT APPLICATION

(21) Application number: 90400298.7

(51) Int. Cl.[5]: C12Q 1/68, C07H 21/04

(22) Date of filing: 02.02.90

(43) Date of publication of application:
07.08.91 Bulletin 91/32

(84) Designated Contracting States:
AT BE CH DE DK ES FR GB GR IT LI LU NL SE

(71) Applicant: N.V. INNOGENETICS S.A.
Industriepark Zwijnaarde 7, Box 4
B-9710 Gent(BE)

(72) Inventor: Rossau, Rudi
Wilgehoevestraat 45
2070 Ekeren(BE)
Inventor: Van Heuverswyn, Hugo
Colmanstraat 62
9288 Laarne(BE)

(74) Representative: Grosset-Fournier, Chantal
Catherine et al
ERNEST GUTMANN-YVES PLASSERAUD S.A.,
67 boulevard Haussmann
F-75008 Paris(FR)

## (54) Hybridization probes for the detection of haemophilus ducreyi strains.

(57) The invention relates to hybridization probes for detecting Haemophilus ducreyi strains.
Representative probes of the invention are characterized by the following nucleotide sequences :

| | |
|---|---|
| CACCCTTTAA TCCGAAGATA TTACG | HD1 |
| AATCAATTTT GCTATTAACA AAACTGCC | HD2 |
| GCTTCGGGCG CCAGTCTTAA AGACCAAC | HD3 |
| CACAAACTCA TCTCTGAGTT CTTCTATGG | HD4 |
| CTCCCCTTTG CAGGTTTGCC GCCC | HD5 |
| GGGTAATAAA TACTAGATTC ATCATCTA | HD6 |
| ATGTATGGGT TATCACTAAC | HD7 |
| TGTTGTTAGT TTAGTACGGC CCACATAT | HD8 |
| CACCCAAGGA GCAAGTCCTT CGTGCT | HD9 |
| CGTTTCTTGA TCAACTCATC CAA | HD10 |
| TGATGTAGCT TCCCAGCTAC | HD11 |




Rank Xerox (UK) Business Services

# HYBRIDIZATION PROBES FOR THE DETECTION OF HAEMOPHILUS DUCREYI STRAINS.

The invention relates to hybridization probes for detecting strains belonging to the species Haemophilus ducreyi. Hereafter, the word strains also encompasses isolates or organisms contained in a biological sample.

Haemophilus ducreyi is a fastidious Gram-negative bacterium which may be involved in serious infections in human beings. Although diagnostic testprocedures for this organism exist, the speed and the specificity of the detection can be considerably improved by using deoxyribonucleic acid (DNA)-probe assays. These DNA probes can, for instance be total genomic DNA, plasmids or synthetic oligonucleotides and these probes may target the genomic DNA or messenger or stable ribonucleic acid (RNA) species present in biological samples. Although not necessary, the use of synthetic oligonucleotides is preferred. The limited length of the oligonucleotides provides extreme specificity; a few mismatches induce a considerable decrease in stability of the probe-target duplex. Oligonucleotides can be rapidly synthesized in great amount, have a long shelf-life, and are easily purified and labeled.

Species-specific probes have been described for a great number of organisms including Haemophilus ducreyi (PARSONS et al., J. Clin. Microbiol. 27 : 1441-1445, 1989). However, species specific probes derived from the ribosomal PNA (rRNA) genes of Haemophilus ducreyi have not been described.

The aim of the invention is to provide with probe sequences deduced from variable regions within the rRNA molecules.

Such probes have an increased sensitivity, because ribosomal rRNA molecules are very abundant in a cell as compared to the genome.

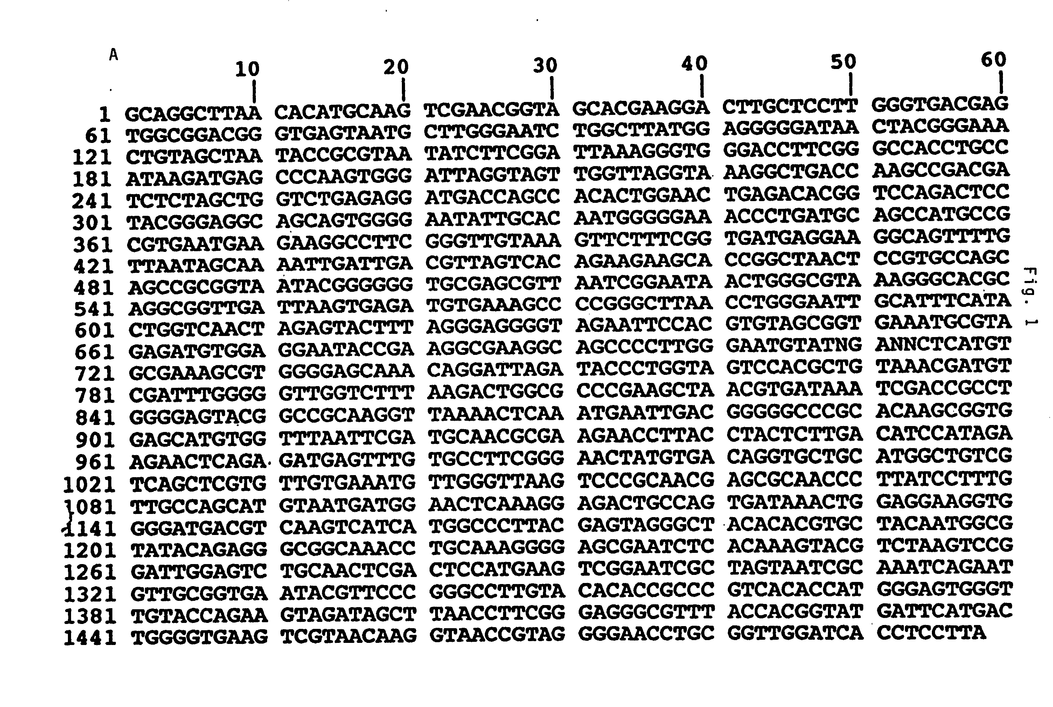

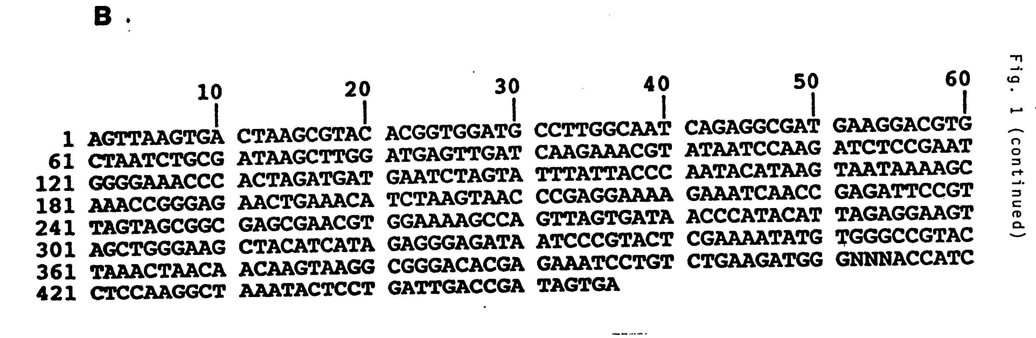

However, because of the high conservation of the rRNA gene, it was unexpected that specific rRNA derived probes could be obtained, which would be specific so as to differentiate the organism concerned from its closest neighbors. That is why for each particular case, the specificity and sensitivity of the probes have to be verified experimentally. For example, according to the invention, a species-specific probe for Haemophilus ducreyi could be unexpectedly found in regions allocated between nucleotides 1209 and 1232 of the 16S rRNA sequence and between nucleotides 271 and 280 of the 23S rRNA sequence (the numbering refers to the numbering in figure 1), whereas no species-specific probes for Neisseria gonorrhoeae could be inferred.

The sequence of the rRNA derived probes is preferably complementary to the rRNA sequence itself, but probes with sequences identical to the rRNA sequence and which subsequently target the DNA molecules, can be used as well for specific detection.

These rRNA-directed probes or their complements can also be used to detect fragments obtained by enzymatical amplification of the target sequence concerned.

Thus an object of the invention is to provide rRNA derived DNA probes for detecting most if not all Haemophilus ducreyi strains.

Another object of the invention is to provide DNA probes for detecting Haemophilus ducreyi strains by a hybridization test, such as a dot-spot, a strand-displacement or a sandwich hybridization test, without resorting to any complementary analysis, such as the Southern-blot analysis.

Still another object of the invention is to provide probes and a simple method for the in vitro diagnosis of Haemophilus ducreyi strains.

"rRNA-related" as used herein refers to the fact that the probed concerned hybridize with sequences normally present in ribosomal RNAs, no matter whether said probes are themselves formed of DNA or RNA fragments, or whether they consist of cloned fragments (in the case of DNA) or of synthetic oligonucleotides.

A hybridization probe of the invention for detecting Haemophilus ducreyi strains contains :

- . either a sequence belonging to a nucleic acid selected from the following groups of nucleic acids and which includes itself of from 15 to the maximum number of nucleotides of the selected nucleic acid,

Group HD1 :

| | |
|---|---|
| CACCCTTTAA TCCGAAGATA TTACG | HD1 |
| CGTAATATCT TCGGATTAAA GGGTG | HD1IC |
| CGUAAUAUCU UCGGAUUAAA GGGUG | HD1ICR |
| CACCCUUUAA UCCGAAGAUA UUACG | HD1R |

Group HD2 :

| | |
|---|---|
| AATCAATTTT GCTATTAACA AAACTGCC | HD2 |
| GGCAGTTTTG TTAATAGCAA AATTGATT | HD2IC |
| GGCAGUUUUG UUAAUAGCAA AAUUGAUU | HD2ICR |
| AAUCAAUUUU GCUAUUAACA AAACUGCC | HD2R |

Group HD3 :

| | |
|---|---|
| GCTTCGGGCG CCAGTCTTAA AGACCAAC | HD3 |
| GTTGGTCTTT AAGACTGGCG CCCGAAGC | HD3IC |
| GUUGGUCUUU AAGACUGGCG CCCGAAGC | HD3ICR |
| GCUUCGGGCG CCAGUCUUAA AGACCAAC | HD3R |

Group HD4 :

| | |
|---|---|
| CACAAACTCA TCTCTGAGTT CTTCTATGG | HD4 |
| CCATAGAAGA ACTCAGAGAT GAGTTTGTG | HD4IC |
| CCAUAGAAGA ACUCAGAGAU GAGUUUGUG | HD4ICR |
| CACAAACUCA UCUCUGAGUU CUUCUAUGG | HD4R |

| | | |
|---|---|---|
| Group HD5 : | | |
| CTCCCCTTTG CAGGTTTGCC GCCC | HD5 |
| GGGCGGCAAA CCTGCAAAGG GGAG | HD5IC |
| GGGCGGCAAA CCUGCAAAGG GGAG | HD5ICR |
| CUCCCCUUUG CAGGUUUGCC GCCC | HD5R |
| Group HD6 : | | |
| GGGTAATAAA TACTAGATTC ATCATCTA | HD6 |
| TAGATGATGA ATCTAGTATT TATTACCC | HD6IC |
| UAGAUGAUGA AUCUAGUAUU UAUUACCC | HD6ICR |
| GGGUAAUAAA UACUAGAUUC AUCAUCUA | HD6R |
| Group HD7 : | | |
| ATGTATGGGT TATCACTAAC | HD7 |
| GTTAGTGATA ACCCATACAT | HD7IC |
| GUUAGUGAUA ACCCAUACAU | HD7ICR |
| AUGUAUGGGU UAUCACUAAC | HD7R |
| Group HD8 : | | |
| TGTTGTTAGT TTAGTACGGC CCACATAT | HD8 |
| ATATGTGGGC CGTACTAAAC TAACAACA | HD8IC |
| AUAUGUGGGC CGUACUAAAC UAACAACA | HD8ICR |
| UGUUGUUAGU UUAGUACGGC CCACAUAU | HD8R |
| Group HD9 : | | |
| CACCCAAGGA GCAAGTCCTT CGTGCT | HD9 |
| AGCACGAAGG ACTTGCTCCT TGGGTG | HD9IC |
| AGCACGAAGG ACUUGCUCCU UGGGUG | HD9ICR |
| CACCCAAGGA GCAAGUCCUU CGUGCU | HD9R |
| Group HD10 : | | |
| CGTTTCTTGA TCAACTCATC CAA | HD10 |
| TTGGATGAGT TGATCAAGAA ACG | HD10IC |
| UUGGAUGAGU UGAUCAAGAA ACG | HD10ICR |
| CGUUUCUUGA UCAACUCAUC CAA | HD10R |
| Group HD11 : | | |
| TGATGTAGCT TCCCAGCTAC | HD11 |
| GTAGCTGGGA AGCTACATCA | HD11IC |
| GUAGCUGGGA AGCUACAUCA | HD11ICR |
| UGAUGUAGCU UCCCAGCUAC | HD11R |

- or a variant sequence which differs from any of the preceding sequences
  . either by addition to or removal from any of their respective extremities of one or several nucleotides,
  . or changing within any of said sequences of one or more nucleotides,
  . or both,

yet provided that in any of the above circumstances said probe still hybridizes with the same RNA or DNA target as the corresponding unmodified sequence.

Probes targeting rRNA are advantageous over probes which target the genomical DNA such as the probe described by Parsons et al., since rRNA is single stranded and consequently directly available for hybridization, and is present in multiple copies in bacterial cells.

In order to obtain the probe sequences of the invention, rRNA genes were enzymatically amplified using the polymerase chain reaction. As primers conserved sequences in the 16S or 23S rRNA were used. The amplified fragments were cloned in a plasmid vector and sequenced using the dideoxy chain termination method. This approach is considerably less tedious and time-consuming than the conventional cloning procedures using genomic banks or selected restriction endonuclease fragments. Although rRNA sequences are more rapidly obtained when the sequencing reactions are performed directly on purified rRNA or PCR fragments without cloning, the sequence information generated from cloned fragments is more accurate and complete. Since the strong secondary structure of rRNA molecules introduces many ambiguities within the nucleotide sequences obtained using the dideoxy chain termination method, it is advisory to sequence both strands, which is not possible with purified rRNA. In contrast to PCR-fragments, cloned rRNA gene fragments can easily be purified in great amounts, which results in clearly readable sequencing ladders. since one mismatch in the probe-sequence may result in useless probes, accuracy is highly preferred over the speed of obtaining rRNA sequences.

In the sequences given in groups HD1 to HD11, the letters mean the following nucleotides :

A : Adenylic residue
C : Cytidylic residue
G : Guanidylic residue
T : Thymidylic residue
U : Uracylic residue

Under the expression "target" is meant a sequence complementary to any of the sequences of groups HD1 to HD11 as herein before defined. This in case where the probe of the invention would comprises nucleic acid elongations on either side or both of said above defined sequences -- e.g. nucleic acid fragments of cloning vector or linker fragments resulting from the cleavage of said probe out of said cloning vector --it is understood that such elongations should be selected such as to avoid the possibility that they could themselves hybridize with any other corresponding complementary nucleic acid sequence outside of the above target in a DNA of any microorganism likely to be tested by the process of this invention as later defined. Such hybridization would be of a parasitical nature and reduce the specificity of the probe.

Preferred probes of the invention consist of nucleic acid fragments formed of any of the sequences of the groups above defined, said fragments containing from 15 to the maximum number of nucleotides of the relevant nucleic acid sequence.

It is understood that in the above nucleotide sequences (and in the other ones referred to hereafter), the left end of the formulae always corresponds to a 5' extremity and the right end to a 3' extremity of the sequence concerned.

When reference is further made therein to a "probe of group 'X'" -- with 'X' from HD1 to HD11 --it should be understood that such probe has a sequence included in one of the nucleic acids belonging to that group as defined above or further defined hereafter.

It is also understood that the word "nucleotide" as used herein refers indistinctly to ribonucleotides and deoxyribonucleotides and modified nucleotides such as inosine unless otherwise specified. The expression "nucleotides" also encompasses those which further comprise modification groups, e.g. chemical modification groups which do not affect their hybridization capabilities. Such modification groups aim, for instance, at facilitating their coupling, either directly or indirectly, with suitable markers or labels for the subsequent detection of the probes so marked or labeled particularly in their hybridization products with the relevant rRNA or DNA strand, e.g. that or those initially contained in a biological sample together with other DNA(s) and/or RNA(s).

For instance, such modification groups are recognizable by antibodies which, in turn, can be recognized specifically by other antibodies, carrying a suitable enzymatic or fluorescent or chemiluminescent label. Possible labeling procedures will further be exemplified later herein.

The invention also relates to probes having any of the sequences defined above and in which some nucleotides are different, provided that the different nucleotide(s) do(es) not alter the specificity of the probes defined above. Some probes may consist of one of the nucleic acids belonging to any of the groups which are set forth above or of part thereof, said probes however including nucleotidic elongation on either sides thereof to the extent that such elongations do no alter the specificity of said probes with the genetic material of Haemophilus ducreyi.

The invention thus provides for probes which are either replicas (those designated by numbers followed by "IC" or 'ICR") in terms of nucleotide sequence of sequences contained in the RNAs or DNAs of most Haemophilus ducreyi strains with occasionally a few insignificant differences in nucleotide sequences or formed of sequences, those designated by bare numbers or by numbers followed by "R", complementary to sequences included in the natural RNAs of Haemophilus ducreyi strains.

More particularly, it should be appreciated that the target sequences in the RNAs or DNAs concerned consist in any of the following successive sequences present in most, if not all, Haemophilus ducreyi strains, subject to possible insignificant natural differences from one strain to another, whereby such natural differences are not likely to affect the hybridization specificity of the probes of this invention which such targets :

CGUAAUAUCU UCGGAUUAAA GGGUG

GGCAGUUUUG UUAAUAGCAA AAUUGAUU

GUUGGUCUUU AAGACUGGCG CCCGAAGC

CCAUAGAAGA ACUCAGAGAU GAGUUUGUG

GGGCGGCAAA CCUGCAAAGG GGAG

UAGAUGAUGA AUCUAGUAUU UAUUACCC

GUUAGUGAUA ACCCAUACAU

AUAUGUGGGC CGUACUAAAC UAACAACA

AGCACGAAGG ACUUGCUCCU UGGGUG

UUGGAUGAGU UGAUCAAGAA ACG

GUAGCUGGGA AGCUACAUCA

The preferred rRNA derived probes are those which are complementary to the natural rRNAs concerned for they hybridize both with said RNAs and the corresponding DNA. Yet, those which have sequences included in said rRNAs therefore which will only hybridize with the relevant natural DNAs and therefore are less sensitive as the preceding ones, are also part of the invention.

The probes according to the invention can be formed by cloning of recombinant plasmids containing inserts including the corresponding nucleotide sequences, if need be cleaving the latter out from the cloned plasmids upon using the adequate nucleases and recovering them, e.g. by fractionation according to molecular weights. The probes according to the invention can be also be synthesized chemically, for instance by the conventional phospho-triester method.

Among the variants defined hereabove are included hybridization probes for detecting Haemophilus ducreyi strains which target one of the sequences defined hereunder or their corresponding complementary sequence, when the hybridization medium or the wash medium or both as appropriate are the following ones :

. hybridization medium : containing about 3 x SSC, (SSC = 0.15 M NaCl, 0.015 M sodium citrate, pH 7.0) about 25 mM of phosphate buffer pH 7.1, 20% deionized formamide 0.02% ficoll, 0.02% bovine serum albumin, 0.02% polyvinylpyrrolidone, and about 0.1 mg/ml sheared, denatured salmon sperm DNA,

. wash medium : containing about 3 x SSC, 25 mM phosphate buffer pH 7.1, and 20% deionized formamide, wherein the target sequence and the corresponding relevant hybridization temperature (HT) and wash temperature (WT) respectively are as follows :

CGUAAUAUCU UCGGAUUAAA GGGUG

HT and/or WT : 45 °C

GGCAGUUUUG UUAAUAGCAA AAUUGAUU

HT and/or WT : 50 °C

GUUGGUCUUU AAGACUGGCG CCCGAAGC

HT and/or WT : 57 °C

CCAUAGAAGA ACUCAGAGAU GAGUUUGU

HT and/or WT : 50 °C

GGGCGGCAAA CCUGCAAAGG GGAG

HT and/or WT : 55 °C

UAGAUGAUGA AUCUAGUAUU UAUUACCC

HT and/or WT : 45 °C

GUUAGUGAUA ACCCAUACAU

HT and/or WT : 40 °C

AUAUGUGGGC CGUACUAAAC UAACAACA

HT and/or WT : 40 °C

AGCACGAAGG ACUUGCUCCU UGGGUG

HT and/or WT : 53 °C

UUGGAUGAGU UGAUCAAGAA ACG

HT and/or WT : 42 °C

GUAGCUGGGA AGCUACAUCA

HT and/or WT : 42 °C.

The invention also relates to a process for detecting Haemophilus ducreyi strains in a biological sample, wherein said process comprises contacting said biological sample in which the nucleic acids (DNAs and RNAs) have been made accessible to hybridization, if need be under suitable denaturation conditions, with a probe of the invention under conditions enabling hybridization between the probe and complementary nucleic acids of the strains, which may be present in the sample, and detecting the hybrids possibly formed.

The process of the invention enables to discriminate Haemophilus ducreyi from any other organism such as yeast, fungi, protozoa, other bacterial strains, human cells which are liable to the be present in the sample in which Haemophilus ducreyi is sought.

The process relates to the detection of Haemophilus ducreyi strains being directly in the sample of after the strain has been cultured.

The detection of a hybrid can be interpreted as meaning that an infection due to Haemophilus ducreyi, was present in the biological sample, when any of the probes of groups HD1 to HD11 is being used respectively.

According to an advantageous embodiment of the invention, in the process for detecting Haemophilus ducreyi strains, the probes used are the ones hybridizing both with DNA globally and RNA of the Haemophilus ducreyi strains, which may be present in the biological sample.

The hybridization conditions can be monitored relying upon several parameters, e.g. hybridization temperature, the nature and concentration of the components of the media, and the temperature under which the hybrids formed are washed.

The hybridization and wash temperature is limited in upper value, according to the probe (its nucleic acid composition, kind and length) and the maximum hybridization or wash temperature of the probes described herein is about 40°C to 57°C.

At higher temperatures duplexing competes with the dissociation (or denaturation) of the hybrid formed between the probe and the target.

A preferred hybridization medium contains about 3 x SSC, (SSC = 0.15 M NaCl, 0.015 M sodium citrate, pH 7.0), about 25 mM of phosphate buffer pH 7.1, and 20% deionized formamide, 0.02 % ficoll, 0.02 % bovine serum albumin, 0.02 % polyvinylpyrrolidone and about 0.1 mg/ml sheared denatured salmon sperm DNA.

A preferred wash medium contains about 3 x SSC, 25 mM phosphate buffer pH 7.1 and 20 % deionized formamide. Other hybridization or wash media can be used as well.

However, when modifications are introduced, be it either in the probes or in the media, the temperatures at which the probes can be used to obtain the required specificity, should be changed according to known relationships, such as those described in the following reference : B.D. HAMES and S.J. HIGGINS, (eds.). Nucleic acid hybridization. A practical approach, IRL Press, Oxford, U.K., 1985.

The process for detecting Haemophilus ducreyi strains generally, according to the invention can be carried out by suitably adjusting the hybridization temperature to a value at which hybridization is specific, and in such a case washing under more stringent conditions is not necessary.

According to another embodiment of the process of the invention, the hybridization temperature needs not necessarily be adjusted to the value at which hybridization is specific and in particular can be lower than the temperature at which hybridization is specific, provided washing is carried out at a temperature corresponding to the value at which hybridization is specific.

In a process embodiment for detecting Haemophilus ducreyi strains (and for distinguishing them from other bacterial taxa) with a probe of group HD1 the hybridization temperature is suitably adjusted to range of about 45° C and/or the wash temperature to range of about 45° C the media being those above defined.

In another process embodiment for detecting Haemophilus ducreyi strains the probe used is anyone of group HD2 above defined, the hybridization temperature is suitably adjusted to range of about 50° C and/or the wash temperature to range of about 50° C, the media being those above defined.

In a further process embodiment for detecting Haemophilus ducreyi strains, the probe used is anyone of group HD3 above defined. The hybridization temperature is suitably adjusted to range of about 57° C and/or the wash temperature to range of about 57° C, the media being those above defined.

In a further process embodiment for detecting Haemophilus ducreyi strains, the probe used is anyone of group HD4 above defined. The hybridization temperature is suitably adjusted to range of about 50° C and/or the wash temperature to range of about 50° C, the media being those above defined.

In a further process embodiment for detecting Haemophilus ducreyi strains, the probe used is anyone of group HD5 above defined. The hybridization temperature is suitably adjusted to range of about 55° C and/or the wash temperature to range of about 55° C, the media being those above defined.

In a further process embodiment for detecting Haemophilus ducreyi strains, the probe used is anyone of group HD6 above defined. The hybridization temperature is suitably adjusted to range of about 45° C and/or the wash temperature to range of about 45° C, the media being those above defined.

In a further process embodiment for detecting Haemophilus ducreyi strains, the probe used is anyone of group HD7 above defined. The hybridization temperature is suitably adjusted to range of about 40° C and/or the wash temperature to range of about 40° C, the media being those above defined.

In a further process embodiment for detecting Haemophilus ducreyi strains, the probe used is anyone of group HD8 above defined. The hybridization temperature is suitably adjusted to range of about 40° C and/or the wash temperature to range of about 40° C, the media being those above defined.

In a further process embodiment for detecting Haemophilus ducreyi strains, the probe used is anyone of group HD9 above defined. The hybridization temperature is suitably adjusted to range of about 53° C and/or the wash temperature to range of about 53° C, the media being those above defined.

In a further process embodiment for detecting Haemophilus ducreyi strains, the probe used is anyone of group HD10 above defined. The hybridization temperature is suitably adjusted to range of about 42° C and/or the wash temperature to range of about 42° C, the media being those above defined.

In a further process embodiment for detecting Haemophilus ducreyi strains, the probe used is anyone of group HD11 above defined. The hybridization temperature is suitably adjusted to range of about 42° C and/or the wash temperature to range of about 42° C, the media being those above defined.

The invention further relates to a kit for detecting specifically Haemophilus ducreyi strains containing :

- at least one probe selected among any of those that are specific for Haemophilus ducreyi as above defined, i.e. a probe of groups HD1, HD2, HD3, HD4, HD5, HD6, HD7, HD8, HD9, HD10, or HD11;
- the buffer or components necessary for producing the buffer enabling an hybridization reaction between these probes and only the DNAs and/or RNAs of a strain of Haemophilus ducreyi to be carried out,
- when appropriate means for detecting the hybrids resulting from the proceeding hybridization.

The probes of the invention can be used in a sandwich hybridization system which enhances the specificity of a nucleic acid probe based assay.

The principle and the use of sandwich hybridizations in a nucleic acid probe based assay have been already described (e.g.: DUNN and HASSEL, Cell, 12 : 23-36; 1977; RANKI et al., Gene, 21 : 77-85; 1983). Although direct hybridization assays have favorable kinetics, sandwich hybridizations are advantageous with respect to a higher signal to noise ratio. Moreover sandwich hybridizations can enhance the specificity of a nucleic acid probe based assay. If properly designed, a sandwich hybridization assay indeed maximizes the specificity of a nucleic acid probe based test when using two probes recognizing two different nucleic acid stretches of one and the same organism. The only demands which must be met are that both probes (i) hybridize to the same nucleic acid molecule of the target organism and (ii) do not hybridize to the same non-target organisms.

For two given probes I and II, the sandwich hybridization system can be described as follows :

Probe n° I hybridizes to nucleic acid from organisms A and B (not with C);

Probe n° II hybridizes to nucleic acid from organisms A and C (not with B).

Since it is absolutely required that both probes hybridize to the target nucleic acid, a detectable signal will be generated only if nucleic acid from organism A is present in the sample. It is obvious that if one of the probes is specific for the organism to be detected, the other probe can be composed of any specific or non-specific sequence provided that it hybridizes to the same target molecule than the first probe.

The probes of the invention -- groups HD1 to HD11 -- can be combined in a sandwich hybridization assay which is highly specific for Haemophilus ducreyi. Hereunder some advantageous combinations, which are specific for Haemophilus ducreyi are given by way of example and not by way of limitation :

- probe of group HD1 ad of corresponding probe of group HD2, HD3, HD4 or HD5;
- probe of group HD2 and a probe of group HD3, HD4 or HD5;
- probe of group HD3 and a probe of group HD4 or HD5;
- probe of group HD4 and a probe of group HD5;
- probe of group HD6 and a probe of group HD7 or HD8;
- probe of group HD7 and a probe of group HD8.

The advantageous approximate hybridization temperature and wash temperature (in °C) for the above mentioned combinations in the media described above are given in the following table :

| | HD1 | HD2 | HD3 | HD4 | HD6 | HD7 |
|---|---|---|---|---|---|---|
| HD2 | 45 | - | - | - | - | - |
| HD3 | 45 | 50 | - | - | - | - |
| HD4 | 45 | 50 | 50 | - | - | - |
| HD5 | 45 | 50 | 55 | 50 | - | - |
| HD7 | - | - | - | - | 40 | - |
| HD8 | - | - | - | - | 40 | 40 |

All these combinations have the 16S or 23S rRNA molecule as target. Combinations between 16S rRNA-and 23S rRNA-derived probes are only possible if the genomic DNA is the target-molecule.

In the sandwich hybridization process the probes can be added simultaneously or not, to the biological sample in which the target DNA or RNA is sought.

The invention also relates to a kit for sandwich hybridization assay, for the detection in vitro of Haemophilus ducreyi strains in a biological sample, said kit containing :

- at least one of the probes or one of the combinations of probes specific for the organisms of interest as above defined.
- the buffer or components necessary for producing the buffer enabling hybridization reaction between these probes and the DNAs and/or RNAs of a strain of Haemophilus ducreyi to be carried out,
- when appropriate means for detecting the hybrids resulting from the preceding hybridization.

The oligonucleotides of the invention can be used either as amplification primers in the polymerase chain reaction technique (PCR; Mullis and Faloona, Methods in Enzymology 155 :335- , 1987.) to generate specific enzymatically amplified fragments and/or as probes to detect fragments amplified between bracketing oligonucleotide primers.

The specificity of a PCR-assisted hybridization assay can be controlled at different levels.

The amplification process or the detection process or both can be specific. The latter case, giving the highest specificity, is preferred. Such a highly specific PCR-assisted test can be developed using the probes of the invention.

CONDITIONS OF THE USE OF PROBES :

The probes of the invention are advantageously labeled. Any conventional label can be used. The probes can be labeled by means of radioactive tracers such as $^{32}P$, $^{35}S$, $^{125}I$, $^{3}H$ and $^{14}C$.

The radioactive labeling can be carried out according to any conventional method such as terminal labeling at the 3' or 5' position with the use of a radio-labeled nucleotide, a polynucleotide kinase (with or without dephosphorylation by a phosphatase), a terminal transferase, or a ligase (according to the extremity to be labeled). One of the probes of the invention can be the matrix for the synthesis of a chain consisting of several radioactive nucleotides or of several radioactive and non radioactive nucleotides.

The probes of the invention can also be prepared by a chemical synthesis using one or several radioactive nucleotides. Another method for radioactive labeling is a chemical iodination of the probes of the invention which leads to the binding of several 125I atoms on the probes.

If one of the probes of the invention is made radioactive to be used for hybridization with a non

radioactive RNA or DNA, the method of detecting hybridization will depend on the radioactive tracer used.

Generally, autoradiography, liquid scintillation, gamma counting or any other conventional method enabling one to detect an ionizing ray issued by the radioactive tracer can be used.

Non radioactive labeling can also be used by associating the probes of the invention with residues having : immunological properties (e.g. antigen or hapten) a specific affinity for some reagents (e.g. ligand) properties providing a detectable enzymatic reaction (e.g. enzyme, co-enzyme, enzyme substrate or substrate taking part in an enzymatic reaction) or physical properties such as fluorescence or emission or absorption of light at any wave length. Antibodies which specifically detect the hybrids formed by the probe and the target can also be used.

A non-radioactive label can be provided when chemically synthesizing a probe of the invention, the adenosine, guanosine, cytidine, thymidine and uracyl residues, thereof being liable to be coupled to other chemical residues enabling the detection of the probe or the hybrids formed between the probe and a complementary DNA or RNA fragment.

However, the nucleotidic sequence of the probe when modified by coupling one or more nucleotides to other chemical residues, would be the same as the nucleotide sequence of one of the probes of the invention.

The invention also relates to processes for detecting by hybridization RNA and/or DNA with the probes of the invention, which have been labeled and can be detected as described above. In this regard, conventional methods of hybridization can be used.

For detecting cells coming from or being themselves living organisms, the RNA and/or DNA of these cells if need be, is made accessible by partial or total lysis of the cells, using chemical or physical processes, and contacted with one or several probes of the invention which can be detected. This contact can be carried out on an appropriate support such as a nitrocellulose, cellulose, or nylon filter in a liquid medium or in solution. This contact can take place under sub-optimal, optimal conditions or under restrictive conditions (i.e. conditions enabling hybrid formation only if the sequences are perfectly homologous on a length of molecule). Such conditions include temperature, concentration of reactants, the presence of substances lowering the optimal temperature of pairing of nucleic acids (e.g. formamide, dimethylsulfoxide and urea) and the presence of substances apparently lowering the reaction volume and/or accelerating hybrid formation (e.g. dextran sulfate, polyethyleneglycol or phenol).

The elimination of probe of the invention which has not hybridized can be carried out by washing with a buffer solution of appropriate ionic strength and at an appropriate temperature, with or without treatment with S1 nuclease or any other enzyme digesting single strand DNA or RNA but not digesting DNA-RNA hybrids or double strand DNA.

In a liquid medium, the hybrids of the probe of the invention paired to the cellular DNA or RNA fragments can be separated from the rest of the liquid medium in different ways, e.g. by chromatography over hydroxyapatite.

Then the hybridized probes are detected by means of the label on the probe.

In order to target the chromosomal DNA fragments carrying the genes coding for the RNA fragments from which the labeled probes of the invention derive, after treating DNA by one or several enzymes and denaturation of DNA fragments (i.e. separation of both chains), one of the probes of the invention is contacted with the DNA fragments under the conditions enabling hybridization and after the time necessary to get to the end of the hybridization, the non-hybridized fragments are separated from the hybridized fragments and the label is detected as it has been described above for the detection of the cells.

Generally speaking, the different probes of the invention can also be contained in recombinant DNA enabling their cloning, if the presence of a heterologous DNA is not a nuisance for the specificity of the probes in the encompassed uses.

Figures 1A and 1B represent the partial nucleotide sequence of the cloned rRNA gene of Haemophilus ducreyi CIP 542.

In this figure, the nucleotide sequence of the upper (sense) strand is shown in its linear conformation from 5' to 3'. More precisely, the linear conformation from 5' to 3' corresponds to the nucleotide sequence of the upper (sense) strand of the rRNA gene, figure 1A corresponding to a partial sequence of the 16S rRNA gene and figure 1B corresponding to a partial sequence of the 23S rRNA gene.

The strain of Haemophilus ducreyi used is Haemophilus ducreyi CIP 542 which is available at Institut Pasteur, Paris. Probes of groups HD1 to HD5, and HD9 are derived from the 16S rRNA gene, and probes of groups HD6, HD7, HD8, HD10 and HD11 are derived from the 23S rRNA gene.

EXAMPLE :

The example hereafter relates to the preparation of the probes of the invention and the experimental results with respect to the specificity and sensitivity of the probes.

The methods used are essentially the same as described by ROSSAU et al., J. Gen. Microbiol.; 135 : 1735-1745, 1989; or in the European patent application n° 8940/045.3 unless otherwise stated.

All methods used, except the enzymatical amplification of rRNA gene fragments, are currently known to people skilled in the art. The enzymatical amplification of rRNA gene fragments of about 500 to 4500 basepairs were obtained by the polymerase chain reaction technique (PCR) performed according to the recommendations given in the "Gene Amp" kit of Perkin Elmer Cetus. As PCR primers oligonucleotides corresponding to conserved or semi-conserved regions in the rRNA molecules were used.

Haemophilus ducreyi, the causative agent of chancroid, is a fastidious Gram-negative bacterium. The culture of this organism is both difficult and insensitive; yet it still is the method of choice for the diagnosis of Haemophilus ducreyi infections. The use of highly specific probes may obviate the culture and increase the sensitivity of the diagnosis.

Part of the rRNA gene of the type strain of Haemophilus ducreyi CIP 542 was enzymatically amplified by the polymerase chain reaction and cloned in a plasmid vector.

The sequence of the insert was obtained by the dideoxy chain termination technique.

From the nucleic acid sequence shown in Fig. 1, the following oligonucleotides were selected and chemically synthesized :

| | |
|---|---|
| CACCCTTTAA TCCGAAGATA TTACG | HD1 |
| AATCAATTTT GCTATTAACA AAACTGCC | HD2 |
| GCTTCGGGCG CCAGTCTTAA AGACCAAC | HD3 |
| CACAAACTCA TCTCTGAGTT CTTCTATGG | HD4 |
| CTCCCCTTTG CAGGTTTGCC GCCC | HD5 |
| GGGTAATAAA TACTAGATTC ATCATCTA | HD6 |
| ATGTATGGGT TATCACTAAC | HD7 |
| TGTTGTTAGT TTAGTACGGC CCACATAT | HD8 |

The oligonucleotides were $^{32}P$-labeled at their 5'ends or tailed at their 3'ends with digoxigeninated UTP using terminal transferase and used as hybridization probes.

As target dot-spotted denatured genomical DNA or lysed cellular material (immobilized on nylon membranes) of a great number of Haemophilus ducreyi strains from different locations and several strains of other bacterial taxa was used. At the appropriate hybridization and wash conditions (see below) the oligonucleotide probes hybridized exclusively to all Haemophilus ducreyi strains tested. The results obtained at the indicated hybridization and wash temperature are summarized in the following table.

| Probe | N° of strains positive/ N° of strains tested: H. ducreyi | N° of strains positive/ N° of strains tested: non-H.ducreyi | Hybridization and washtemp.(°C) |
|---|---|---|---|
| HD1 | 70/70 | 0/15 | 45 |
| HD2 | 85/85 | 0/15 | 50 |
| HD3 | 89/89 | 0/15 | 57 |
| HD4 | 51/51 | 0/13 | 50 |
| HD5 | 41/41 | 0/13 | 55 |
| HD6 | 68/68 | 0/15 | 45 |
| HD7 | 70/70 | 0/14 | 40 |
| HD8 | 87/87 | 0/15 | 40 |

The hybridization-mixture was either 3 X SSC, 25 mM potassium phosphate buffer, pH 7, deionized formamide (20 %, v/v), Ficoll (0.02%, w/v), bovine serum albumin (0.02%, w/v), polyvinylpyrrolidone (0.02%,

w/v) and sheared, denatured salmon sperm DNA (0.1 mg/ml-1) or the solution given in the protocol sheet of the DNA labeling and detection kit nonradioactive (Boehringer Mannheim) except that 3 X SSC (1 X SSC is : 0.15 M NaCl, 0.015 M sodium citrate, pH 7.0) in stead of 5 X SSC was used and formamide was added up to 20 % (v/v). The wash-solution contained 3 X SSC, 20% formamide and 25 mM phosphate buffer pH 7.1.

The non-Haemophilus ducreyi strains tested are listed below :

Escherichia coli MC 1061
Escherichia coli B
Actinobacillus actinomycetemcomitans NCTC 9710
Actinobacillus lignieresii NCTC 4189
Haemophilus aphrophilus NCTC 5906
Haemophilus influenza NCTC 8143
Histophilus ovis HIM 896-7
Pasteurella multocida NCTC 10322
Branhamella catarrhalis ITG 4197
Comamonas testosteroni ATCC 17407
Oligella urethralis LMG 6227
Neisseria gonorrhoeae ITG 4437
Campylobacter jejuni CCUG 11284
Acinetobacter calcoaceticus ATCC 23055
Unidentified strain ITG 3565

## Claims

**1.** Probe for detecting one or more Haemophilus ducreyi strains, containing :

- either a sequence belonging to a nucleic acid selected from the following group of nucleic acids and which includes itself of from 15 to the maximum number of nucleotides of the selected nucleic acid :

Group HD1 :

| | |
|---|---|
| CACCCTTTAA TCCGAAGATA TTACG | HD1 |
| CGTAATATCT TCGGATTAAA GGGTG | HD1IC |
| CGUAAUAUCU UCGGAUUAAA GGGUG | HD1ICR |
| CACCCUUUAA UCCGAAGAUA UUACG | HD1R |

Group HD2 :

| | |
|---|---|
| AATCAATTTT GCTATTAACA AAACTGCC | HD2 |
| GGCAGTTTTG TTAATAGCAA AATTGATT | HD2IC |
| GGCAGUUUUG UUAAUAGCAA AAUUGAUU | HD2ICR |
| AAUCAAUUUU GCUAUUAACA AAACUGCC | HD2R |

Group HD3 :

| | |
|---|---|
| GCTTCGGGCG CCAGTCTTAA AGACCAAC | HD3 |
| GTTGGTCTTT AAGACTGGCG CCCGAAGC | HD3IC |
| GUUGGUCUUU AAGACUGGCG CCCGAAGC | HD3ICR |
| GCUUCGGGCG CCAGUCUUAA AGACCAAC | HD3R |

Group HD4 :

| | |
|---|---|
| CACAAACTCA TCTCTGAGTT CTTCTATGG | HD4 |
| CCATAGAAGA ACTCAGAGAT GAGTTTGTG | HD4IC |
| CCAUAGAAGA ACUCAGAGAU GAGUUUGUG | HD4ICR |
| CACAAACUCA UCUCUGAGUU CUUCUAUGG | HD4R |

Group HD5 :

| | |
|---|---|
| CTCCCCTTTG CAGGTTTGCC GCCC | HD5 |
| GGGCGGCAAA CCTGCAAAGG GGAG | HD5IC |
| GGGCGGCAAA CCUGCAAAGG GGAG | HD5ICR |
| CUCCCCUUUG CAGGUUUGCC GCCC | HD5R |

Group HD6 :

| | |
|---|---|
| GGGTAATAAA TACTAGATTC ATCATCTA | HD6 |
| TAGATGATGA ATCTAGTATT TATTACCC | HD6IC |

| | |
|---|---|
| UAGAUGAUGA AUCUAGUAUU UAUUACCC | HD6ICR |
| GGGUAAUAAA UACUAGAUUC AUCAUCUA | HD6R |
| Groupe HD7 : | |
| ATGTATGGGT TATCACTAAC | HD7 |
| GTTAGTGATA ACCCATACAT | HD7IC |
| GUUAGUGAUA ACCCAUACAU | HD7ICR |
| AUGUAUGGGU UAUCACUAAC | HD7R |
| Group HD8 : | |
| TGTTGTTAGT TTAGTACGGC CCACATAT | HD8 |
| ATATGTGGGC CGTACTAAAC TAACAACA | HD8IC |
| AUAUGUGGGC CGUACUAAAC UAACAACA | HD8ICR |
| UGUUGUUAGU UUAGUACGGC CCACAUAU | HD8R |
| Group HD9 : | |
| CACCCAAGGA GCAAGTCCTT CGTGCT | HD9 |
| AGCACGAAGG ACTTGCTCCT TGGGTG | HD9IC |
| AGCACGAAGG ACUUGCUCCU UGGGUG | HD9ICR |
| CACCCAAGGA GCAAGUCCUU CGUGCU | HD9R |
| Group HD10 : | |
| CGTTTCTTGA TCAACTCATC CAA | HD10 |
| TTGGATGAGT TGATCAAGAA ACG | HD10IC |
| UUGGAUGAGU UGAUCAAGAA ACG | HD10ICR |
| CGUUUCUUGA UCAACUCAUC CAA | HD10R |
| Group HD11 : | |
| TGATGTAGCT TCCCAGCTAC | HD11 |
| GTAGCTGGGA AGCTACATCA | HD11IC |
| GUAGCUGGGA AGCUACAUCA | HD11ICR |
| UGAUGUAGCU UCCCAGCUAC | HD11R |

- or a variant sequence which distinguishes of any of the preceding sequences :
  * either by addition to or removal from any of their respective extremities of one or several nucleotides;
  * or changing within any of said sequences of one or more nucleotides;
  * or both;

yet provided that in any of the above circumstances said probe still hybridizes with the same RNA or DNA target as the corresponding unmodified sequence.

2. Process for detecting Haemophilus ducreyi strains in a biological sample, wherein said process comprises contacting said biological sample in which the nucleic acids (DNAs and/or RNAs) of the strains have been made accessible to hybridization, if need be, under suitable denaturation conditions with a probe according to any one of claim 1 under conditions enabling hybridization between the probe and complementary nucleic acids of the Haemophilus ducreyi strains, which may be present in the sample, and detecting the hybrids possibly formed.

3. Process for detecting Haemophilus ducreyi, according to claim 2, wherein the preferred hybridization medium or the wash medium or both as appropriate are the following ones :
the hybridization medium : containing about 3 x SSC, (SSC = 0.15 M NaCl, 0.015 M sodium citrate, pH 7.0) about 25 mM of phosphate buffer pH 7.1, 20 % deionized formamide 0.02 % ficoll, 0.02 % bovine serum albumin, 0.02 % polyvinylpyrrolidone, and about 0.1 mg/ml sheared, denatured salmon sperm DNA,
the wash medium : containing about 3 x SSC, 25 mM phosphate buffer pH 7.1, and 20 % deionized formamide, wherein the target sequence and the corresponding relevant hybridization temperature (HT) and wash temperature (WT) respectively are as follows :
CGUAAUAUCU UCGGAUUAAA GGGUG
HT and/or WT : 45 °C
GGCAGUUUUG UUAAUAGCAA AAUUGAUU
HT and/or WT : 50 °C
GUUGGUCUUU AAGACUGGCG CCCGAAGC
HT and/or WT : 57 °C
CCAUAGAAGA ACUCAGAGAU GAGUUUGU
HT and/or WT : 50 °C
GGGCGGCAAA CCUGCAAAGG GGAG
HT and/or WT : 55 °C
UAGAUGAUGA AUCUAGUAUU UAUUACCC
HT and/or WT : 45 °C
GUUAGUGAUA ACCAUACAU
HT and/or WT : 40 °C
AUAUGUGGGC CGUACUAAAC UAACAACA
HT and/or WT : 40 °C
AGCACGAAGG ACUUGCUCCU UGGGUG
HT and/or WT : 53 °C
UUGGAUGAGU UGAUCAAGAA ACG
HT and/or WT : 42 °C
GUAGCUGGGA AGCUACAUCA
HT and/or WT : 42 °C

4. Process for detecting Haemophilus ducreyi strains from other organisms in a biological sample, according to claim 2 or 3, wherein the probes used are the ones hybridizing both with DNA and RNA of Haemophilus ducreyi strains which may be present in the biological sample.

5. Process for detecting Haemophilus ducreyi in a biological sample, according to anyone of claims 2 to 4, wherein
- the hybridization medium contains about 3 x SSC, (SSC = 0.15 M NaCl, 0.015 M sodium citrate, pH 7.0) about 25 mM of phosphate buffer pH 7.1, 20 % deionized formamide 0.02 % ficoll, 0.02 % bovine serum albumin, 0.02 % polyvinylpyrrolidone, and about 0.1 mg/ml sheared, denatured salmon sperm DNA, and/or
- the wash medium contains about 3 x SSC, 25 mM phosphate buffer pH 7.1, and 20 % deionized formamide,

and wherein the probe used is
anyone of the probes HD1, HD1IC, HD1ICR, HD1R of claim 1, the hybridization temperature being suitably adjusted to the range of about 45 °C and/or the wash temperature to the range of about 45 °C, or anyone of the probes HD2, HD2IC, HD2ICR, HD2R of claim 1, the hybridization temperature being suitably adjusted to the range of about 50 °C and/or the wash temperature to the range of about 50 °C, or anyone of the probes HD3, HD3IC, HD3ICR, HD3R of claim 1, the hybridization temperature being suitably adjusted to the range of about 57 °C and/or the wash temperature to the range of about 57 °C, or anyone of the probes HD4, HD4IC, HD4ICR, HD4R of claim 1, the hybridization temperature being suitably adjusted to the range of about 50 °C and/or the wash temperature to the range of about 50 °C, or anyone of the probes HD5, HD5IC, HD5ICR, HD5R of claim 1, the hybridization temperature being suitably adjusted to the range of about 55 °C and/or the wash temperature to the range of about 55 °C, or anyone of the probes HD6, HD6IC, HD6ICR, HD6R of claim 1, the hybridization temperature being suitably adjusted to the range of about 45 °C and/or the wash temperature to the range of about 45 °C, or anyone of the probes HD7, HD7IC, HD7ICR, HD7R of claim 1, the hybridization temperature being

suitably adjusted to the range of about 40° C and/or the wash temperature to the range of about 40° C, or anyone of the probes HD8, HD8IC, HD8ICR, HD8R of claim 1, the hybridization temperature being suitably adjusted to the range of about 40° C and/or the wash temperature to the range of about 40° C, or anyone of the probes HD9, HD9IC, HD9ICR, HD9R of claim 1, the hybridization temperature being suitably adjusted to the range of about 53° C and/or the wash temperature to the range of about 53° C, or anyone of the probes HD10, HD10IC, HD10ICR, HD10R of claim 1, the hybridization temperature being suitably adjusted to the range of about 42° C and/or the wash temperature to the range of about 42° C, or anyone of the probes HD11, HD11IC, HD11ICR, HD11R of claim 1, the hybridization temperature being suitably adjusted to the range of about 42° C and/or the wash temperature to the range of about 42° C.

**6.** Process for detecting Haemophilus ducreyi strains from other organisms, wherein said process comprises contacting said biological sample, in which the nucleic acids (DNAs and/or RNAs) have been made accessible to hybridization, if need be, under suitable denaturation conditions, with two probes, targeting the same nucleic acid molecule, and of which at least one is specific for Haemophilus ducreyi and which is selected from any one of the probes of claim 1, whenever required under hybridization and washing conditions adjusted such as to ensure specific hybridization with complementary nucleic acids of the Haemophilus ducreyi strains, which may be present in the sample, yet not with complementary DNA or RNA of other organisms and detecting the hybrids possibly formed.

**7.** Kit for the detection in vitro of a large number, preferably all Haemophilus ducreyi strains in a biological sample, said kit containing :

- at least one probe selected among any of those according to claim 1;
- the buffer or components necessary for producing the buffer enabling hybridization reaction between these probes and the DNAs and/or RNAs of a large number, preferably all strains of Haemophilus ducreyi to be carried out;
- when appropriate means for detecting the hybrids resulting from the preceding hybridization.

**8.** Kit for sandwich hybridization assay, for the detection in vitro of Haemophilus ducreyi strains in a biological sample, said kit containing :

- at least two probes, targeting the same nucleic acid molecule, and of which at least one is specific for Haemophilus ducreyi and which is selected from any one of the probes of claim 1,
- the buffer or components necessary for producing the buffer enabling hybridization reaction between these probes and the DNAs and/or RNAs of a strain of Haemophilus ducreyi to be carried out,
- when appropriate means for detecting the hybrids resulting from the preceding hybridization.

**9.** Process for detecting Haemophilus ducreyi strains from other organisms using the polymerase chain reaction technique in which at least one of the probes of claim 1 is used as amplification primer and/or detection probe of the amplified product.

Fig. 1

A

```
              10         20         30         40         50         60
               |          |          |          |          |          |
   1 GCAGGCTTAA CACATGCAAG TCGAACGGTA GCACGAAGGA CTTGCTCCTT GGGTGACGAG
  61 TGGCGGACGG GTGAGTAATG CTTGGGAATC TGGCTTATGG AGGGGGATAA CTACGGGAAA
 121 CTGTAGCTAA TACCGCGTAA TATCTTCGGA TTAAAGGGTG GGACCTTCGG GCCACCTGCC
 181 ATAAGATGAG CCCAAGTGGG ATTAGGTAGT TGGTTAGGTA AAGGCTGACC AAGCCGACGA
 241 TCTCTAGCTG GTCTGAGAGG ATGACCAGCC ACACTGGAAC TGAGACACGG TCCAGACTCC
 301 TACGGGAGGC AGCAGTGGGG AATATTGCAC AATGGGGGAA ACCCTGATGC AGCCATGCCG
 361 CGTGAATGAA GAAGGCCTTC GGGTTGTAAA GTTCTTTCGG TGATGAGGAA GGCAGTTTTG
 421 TTAATAGCAA AATTGATTGA CGTTAGTCAC AGAAGAAGCA CCGGCTAACT CCGTGCCAGC
 481 AGCCGCGGTA ATACGGGGGG TGCGAGCGTT AATCGGAATA ACTGGGCGTA AAGGGCACGC
 541 AGGCGGTTGA TTAAGTGAGA TGTGAAAGCC CCGGGCTTAA CCTGGGAATT GCATTTCATA
 601 CTGGTCAACT AGAGTACTTT AGGGAGGGGT AGAATTCCAC GTGTAGCGGT GAAATGCGTA
 661 GAGATGTGGA GGAATACCGA AGGCGAAGGC AGCCCCTTGG GAATGTATNG ANNCTCATGT
 721 GCGAAAGCGT GGGGAGCAAA CAGGATTAGA TACCCTGGTA GTCCACGCTG TAAACGATGT
 781 CGATTTGGGG GTTGGTCTTT AAGACTGGCG CCCGAAGCTA ACGTGATAAA TCGACCGCCT
 841 GGGGAGTACG GCCGCAAGGT TAAAACTCAA ATGAATTGAC GGGGGCCCGC ACAAGCGGTG
 901 GAGCATGTGG TTTAATTCGA TGCAACGCGA AGAACCTTAC CTACTCTTGA CATCCATAGA
 961 AGAACTCAGA GATGAGTTTG TGCCTTCGGG AACTATGTGA CAGGTGCTGC ATGGCTGTCG
1021 TCAGCTCGTG TTGTGAAATG TTGGGTTAAG TCCCGCAACG AGCGCAACCC TTATCCTTTG
1081 TTGCCAGCAT GTAATGATGG AACTCAAAGG AGACTGCCAG TGATAAACTG GAGGAAGGTG
1141 GGGATGACGT CAAGTCATCA TGGCCCTTAC GAGTAGGGCT ACACACGTGC TACAATGGCG
1201 TATACAGAGG GCGGCAAACC TGCAAAGGGG AGCGAATCTC ACAAAGTACG TCTAAGTCCG
1261 GATTGGAGTC TGCAACTCGA CTCCATGAAG TCGGAATCGC TAGTAATCGC AAATCAGAAT
1321 GTTGCGGTGA ATACGTTCCC GGGCCTTGTA CACACCGCCC GTCACACCAT GGGAGTGGGT
1381 TGTACCAGAA GTAGATAGCT TAACCTTCGG GAGGGCGTTT ACCACGGTAT GATTCATGAC
1441 TGGGGTGAAG TCGTAACAAG GTAACCGTAG GGGAACCTGC GGTTGGATCA CCTCCTTA
```

Fig. 1 (continued)

**B.**

```
                10         20         30         40         50         60
                 |          |          |          |          |          |
  1 AGTTAAGTGA CTAAGCGTAC ACGGTGGATG CCTTGGCAAT CAGAGGCGAT GAAGGACGTG
 61 CTAATCTGCG ATAAGCTTGG ATGAGTTGAT CAAGAAACGT ATAATCCAAG ATCTCCGAAT
121 GGGGAAACCC ACTAGATGAT GAATCTAGTA TTTATTACCC AATACATAAG TAATAAAAGC
181 AAACCGGGAG AACTGAAACA TCTAAGTAAC CCGAGGAAAA GAAATCAACC GAGATTCCGT
241 TAGTAGCGGC GAGCGAACGT GGAAAAGCCA GTTAGTGATA ACCCATACAT TAGAGGAAGT
301 AGCTGGGAAG CTACATCATA GAGGGAGATA ATCCCGTACT CGAAAATATG TGGGCCGTAC
361 TAAACTAACA ACAAGTAAGG CGGGACACGA GAAATCCTGT CTGAAGATGG GNNNACCATC
421 CTCCAAGGCT AAATACTCCT GATTGACCGA TAGTGA
```

European Patent Office

# EUROPEAN SEARCH REPORT

Application Number

EP 90 40 0298

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 272 009 (HOGEN et al.)<br>* Abstract; claims 1-34 *<br>--- | 1 | C 12 Q 1/68<br>C 07 H 21/04 |
| A | EP-A-0 337 896 (N.V. INNOGENETICS)<br>* Pages 2,16-18 *<br>--- | 1-8 | |
| D,A | BIOLOGICAL ABSTRACTS, vol. 88, 1989, abstract no. 50919, Philadelphia, PA, US; L.M. PARSONS et al.: "DNA probes for the identification of Haemophilus ducreyi", & J. CLIN. MICROBIOL. 22(7): 1441-1445. 1989<br>* Whole abstract *<br>----- | 1 | |
| | | | TECHNICAL FIELDS SEARCHED (Int. Cl.5) |
| | | | C 12 Q |

The present search report has been drawn up for all claims

| Place of search | Date of completion of the search | Examiner |
|---|---|---|
| THE HAGUE | 01-10-1990 | OSBORNE H.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P0401)